# EUROPÄISCHE PATENTSCHRIFT

(11) **EP 3 119 355 B1**
(45) Veröffentlichungstag und Bekanntmachung des Hinweises auf die Patenterteilung: **11.04.2018**
(21) Anmeldenummer: 15705790.2
(22) Anmeldetag: 12.02.2015
(51) Int. Cl.: A61F 5/058, A61F 5/10

(54) **KOPPELBARE STÜTZSCHIENE**
COUPLEABLE SUPPORT SPLINT
RAIL DE SUPPORT POUVANT ÊTRE ACCOUPLÉ

(30) Priorität: 20.03.2014 DE 102014004261
(43) Veröffentlichungstag der Anmeldung: 25.01.2017
(73) Patentinhaber: Bauerfeind AG, 07937 Zeulenroda-Triebes (DE)
(72) Erfinder: BECK, André, 99192 Apfelstädt (DE)
(74) Vertreter: Schwahn, Hartmut
(86) Internationale Anmeldenummer: PCT/EP2015/052949
(87) Internationale Veröffentlichungsnummer: WO 2015/139891

(56) Entgegenhaltungen:
- US-A- 1 837 691
- US-A- 2 573 715
- US-A- 3 913 570
- US-A1- 2005 027 223

## Beschreibung

Die Erfindung betrifft medizinische Hilfsmittel, besonders Orthesen zur Stützung von Körperteilen, besonders eine Gelenkstütze zur Stützung oder Fixierung gelenkig verbundener Knochenglieder, wie Finger oder Wirbelsäule. Die Gelenkstütze enthält mindestens eine Stützschiene, die in Segmente gegliedert ist, welche an die gelenkig verbundenen Knochenglieder anlegbar sind. Gemäß der Erfindung ist diese segmentierte Stützschiene mittels Versteifungselementen in Längsrichtung versteifbar und mittels Querkoppelelementen mit benachbarten gleichartigen Stützschienen verkoppelbar.

Gelenkig verbundene Knochenglieder des Körpers, zum Beispiel die Finger der Hand, der Daumen sowie die Zehenglieder, aber auch Abschnitte der Wirbelsäule, besonders Halswirbelsäule und Lendenwirbelsäule, müssen aus medizinischen Gründen nach Trauma oder Verschleiß gestützt, das heißt in ihrer mechanischen Funktion und Beweglichkeit unterstützt, oder aber dauerhaft oder zeitweise ruhiggestellt, das heißt fixiert werden. Eine Fixierung der Gelenkverbindung ist vor allem bei Brüchen, Kapselrissen oder Sehnenverletzungen sowie auch bei entzündlichen Vorgängen und ähnlichen Gelenkerkrankungen erforderlich. Dazu soll die natürliche Beweglichkeit, besonders die Gelenkigkeit der Knochenglieder zueinander teilweise oder vollständig unterbunden werden. Es sind Bandagen oder Orthesen als medizinische Hilfsmittel für bekannt, welche besonders in Form von Stützplatten, Stützschienen und/oder Pelotten an den gelenkigen Knochengliedern, beispielsweise den Fingern, angelegt werden. In der einfachen Form sind dies meist aus kalt verformbaren Metallschienen gebildete Vorrichtungen, die mittels Bandagen oder flexiblen oder elastischen Textilkörpern an dem Gelenk gehalten werden. Nachteilig ist dabei, dass eine medizinisch notwendige stufenweise Rückführung eines zunächst zum Beispiel vollständig fixierten Gelenks in die Beweglichkeit mit diesen herkömmlichen Stützorthesen nicht gewährleistet werden kann, da entweder eine vollständige Blockierung des Gelenks oder aber lediglich eine mechanische Unterstützung der Beweglichkeit als Alternativen zur Verfügung stehen. Besonders sind bekannte Orthesen dieser Gattung nicht ausreichend an den jeweils geforderten individuellen Grad der Stützung oder Fixierung des Gelenks anpassbar, vor allem bei der Stützung mehrgliedriger Körperglieder wie Finger oder Teile der Wirbelsäule.
Daneben ist es, besonders im Bereich der Handorthesen zur Stützung einzelner oder mehrerer Fingerglieder aus medizinischer Sicht erforderlich, je nach Krankheitsbild gezielt nur einzelne Fingerglieder der Hand mechanisch zu stützen oder zu fixieren. Beispielsweise soll das Fingergrundgelenk eines Fingers blockiert werden, das Fingerendgelenk hingegen nur mechanisch unterstützt, aber im Grunde beweglich bleiben. Weiter kann es erforderlich sein, dass im Fingerverband ein einziger erkrankter Finger mechanisch über die benachbarten gesunden Finger gestützt werden soll, wobei in einem akuten Stadium der Erkrankung der erkrankte Finger vollständig fixiert werden, zu einem späteren Zeitpunkt das erkrankte Fingergelenk aber zunehmend ein Bewegungstraining über die benachbarten beweglichen gesunden Finger erfahren soll.

Aus der US 2005/007223 A1, die als nächstliegender Stand der Technik angesehen werden kann, ist eine segmentierte Gelenkschiene zur Stützung gelenkig verbundener Knochenglieder bekannt, wobei die Segmente über Versteifungselemente in Längsrichtung gekoppelt sind. Aus der US 3,913,570 A sind aus Teilschienen zusammensetzbare und mit Kopplungsmitteln verkoppelbare Gelenkschienen für Arme oder Beine bekannt.

Die Erfindung hat sich zur Aufgabe gemacht, eine Orthese, besonders eine Hand- und Fingerorthese, bereitzustellen, welche die vorgenannten Probleme lösen kann.

Die Erfindung stellt dazu ein modulares System, besonders zur Anwendung als oder in einer Hand- und Fingerorthese bereit, welches durch einfache Eingriffe des behandelten Arztes, des Patienten und insbesondere des Orthopädietechnikers auf die jeweiligen Gegebenheiten der Erkrankung und die erforderlichen Maßnahmen angepasst werden kann und so die genannten Anforderungen erfüllt.

Gegenstand der Erfindung ist eine Gelenkstütze zur Stützung gelenkig verbundener Knochenglieder, mit mindestens einer Stützschiene, welche in mindestens zwei Segmente strukturiert ist, wobei jedes Segment einem Knochenglied zuordenbar ist und wobei die Stützschiene je Segment mindestens eine Führung zur Aufnahme eines Versteifungselements zur Längskopplung der mindestens zwei Segmente und Kopplungsmittel zur Querkopplung dieser Stützschiene mit mindestens einer weiteren, dazu benachbarten Stützschiene aufweist.

Bevorzugt sind mindestens zwei miteinander quer verkoppelbare gleichartige Stützschienen vorhanden.

Bevorzugt ist als Kopplungsmittel mindestens eine Rastnase an mindestens einen Segment der einen Stützschiene zum Eingriff in eine Nut an einem Segment der benachbarten Stützschiene ausgebildet.

Alternativ bevorzugt ist als Kopplungsmittel eine Führung an mindestens einem Segment der einen Stützschiene zur Aufnahme eines Querkoppelelements zum Eingriff in eine Führung an einem Segment der benachbarten Stützschiene ausgebildet.

Besonders ist diese Führung als mindestens ein Bügel auf der Stützschiene ausgebildet. Alternativ ist die Führung als Kanal innerhalb der Stützschiene ausgebildet.

Bevorzugt sind die Segmente durch Einkerbung und/oder Materialausdünnung an der Schiene voneinander getrennt.

Gegenstand der Erfindung ist auch eine Handorthese, enthaltend die hierin beschriebene erfindungsgemäße Gelenkstütze, wobei die Knochenglieder Fingerknochen sind.

### Detaillierte Darstellung der Erfindung

Gegenstand der Erfindung ist primär eine Gelenkstütze zur Stützung gelenkig verbundener Knochenglieder, welche mindestens eine segmentierte Stützschiene aufweist. Erfindungsgemäß ist diese Stützschiene in mindestens zwei Segmente strukturiert, wobei besonders jedes Segment einem, besonders jeweils genau einem, zu stützenden Knochenglied zuordenbar ist.

Die Segmente sind insbesondere voneinander durch Materialverdünnung oder Einkerbung des Stützschienengrundmatierials zueinander flexibel oder gelenkig ausgebildet, um insbesondere dem natürlichen Gelenkverlauf der zuordenbaren gelenkigen Knochengliedern zu folgen. So kann vor allem eine die Gelenkbewegung unterstützende Funktion der Stützschiene erreicht werden.

Die Erfindung sieht aber auch vor, dass die Stützschiene, insbesondere je Segment, mindestens eine Führung zur Aufnahme eines Versteifungselements aufweist. Erfindungsgemäß dient dabei ein in die Führung einsetzbares Versteifungselement der mechanischen Gelenkkopplung dieser mindestens zwei, insbesondere benachbarten, Segmente dieser Stützschiene. In einer Variante sind mehr als zwei, besonders alle an der Stutzschiene jeweils vorhandenen Segmente über mindestens ein Versteifungselement verkoppelbar oder verkoppelt.

Die erfindungsgemäß vorgesehenen Führungen an den Segmenten dienen insbesondere dazu, ein Versteifungselement aufzunehmen, welches mindestens eine der Segmentgrenzen überbrückt, um die Beweglichkeit der Stützschiene an dieser Segmentgrenze zu reduzieren oder vollständig zu unterdrücken. Typischerweise kann als Versteifungselement ein Materialstreifen eingelegt werden, welcher aus einem starren, unelastischen Material gefertigt ist. Bevorzugt dient als Versteifungselement ein kalt verformbarer Metallstreifen. Alternativ ein insbesondere thermoverformbarer Kunststoffstreifen. Durch die Verformbarkeit des Versteifungselements können die beiden an der Stützschiene verbundenen Segmente auch in einer bestimmten vorgegebenen Winkelstellung fixiert werden. Versteifungselemente aus Metall und thermoverformbarem Kunststoff sind im Bereich der Orthopädie an sich bekannt.

Je nach medizinischer Indikation oder Therapieverlauf kann beispielsweise ein Versteifungselement, was zunächst in die Stützschiene eingelegt wurde, um die Beweglichkeit der gelenkig verbundenen Knochenglieder zu blockieren, zu einem späteren Zeitpunkt einfach herausgenommen werden, um nun eine Bewegung der gelenkig verbundenen Knochenglieder zu ermöglichen.

Besonders ist die mindestens eine Führung zur Aufnahme eines Versteifungselements zur Längskopplung der mindestens zwei Segmente als Bügel, welche auf den Grundkörper der Stützschiene aufgesetzt sind, ausgebildet. In einer alternativen oder zusätzlichen Ausgestaltung ist in dem Grundkörper der Stützschiene mindestens ein Kanal ausgebildet, worin das Versteifungselement aufgenommen und geführt wird. Zusätzlich kann an der Spitze der Stützschiene und/oder im Bereich des Endes jedes Segments der Stützschiene eine insbesondere federnde Rastung vorgesehen sein, welche das eingesetzte Versteifungselement gegen Verschieben blockiert und damit insbesondere gegen ein Herausfallen des Versteifungselementes schützt.

In einer besonderen Variante dieser Ausgestaltung ist dabei vorgesehen, in mehreren an der Stützschiene ausgebildeten Segmenten diese federnde Rastung vorzusehen, wobei, je nach Länge des eingesetzten Versteifungselements, nur die am Ende des Versteifungselements lokalisierte federnde Rastung das Versteifungselement blockiert, während diejenigen federnden Rastungen, welche sich innerhalb der Erstreckung des Versteifungselements, das heißt besonders unterhalb des Versteifungselements an dem überbrückten Segment befinden, durch die federnde Wirkung nicht unmittelbar mit dem Ende des Versteifungselements in Eingriff kommen, sondern zurückgeklappt im Grundkörper der Stützschiene verbleiben. Auf diese Weise ist eine individuelle Kürzbarkeit des Versteifungselements unabhängig von der Gesamtlänge und Gesamtanzahl der Segmente an dieser Stützschiene möglich, wobei gleichzeitig auch die gekürzten Versteifungselemente mittels der federnden Rastnasen gegen Verschieben blockiert werden.

Die Erfindung sieht weiter vor, dass die Stützschiene Mittel aufweist, welche eine Querverkopplung dieser Stützschiene mit mindestens einer weiteren, insbesondere gleichartig aufgebauten, benachbarten Stützschiene ermöglicht. So kann insbesondere im Zusammenhang mit einer Fingerorthese eine mechanische Verkopplung der Stützschienen und damit der zu stützenden benachbarten Finger ermöglicht werden. Bevorzugt ist dabei vorgesehen, dass diese Orthese zur Stützung der Finger im Wesentlichen modular aus gleichartigen Stützschienen aufgebaut ist. Erfindungsgemäß weist die Stützschiene also, insbesondere je Segment, mindestens ein Kopplungsmittel auf, welches der mechanischen Querkopplung dieser Stützschiene mit mindestens einer weiteren dazu, insbesondere unmittelbar, benachbarten Stützschiene dient. Besonders ist dabei vorgesehen, dass diese benachbarten quer verkoppelbaren Stützschienen im Wesentlichen gleichartig aufgebaut sind. Das heißt besonders, dass auch die benachbarte weitere Stützschiene in mindestens zwei Segmente strukturiert ist und mindestens eine entsprechende Führung zur Aufnahme eines Versteifungselements zur Längskopplung der mindestens zwei Segmente besitzt. Daneben weist die benachbarte weitere Stützschiene besonders auch, insbesondere je Segment, mindestens ein entsprechendes Kopplungsmittel auf, welches der mechanischen Querkopplung mit der benachbarten Stützschiene dient.

In besonderer Ausgestaltung kann die Querverkoppelbarkeit benachbarter Stützschienen untereinander durch die erfindungsgemäß vorgesehenen Kopplungsmittel auf bestimmte Segmente der Stützschiene beschränkt sein. Dazu ist besonders vorgesehen, dass die Kopplungsmittel selektiv pro Segment ausgebildet sind und selektiv pro Segment durch einfachen Eingriff unterbunden werden kann. Dies geschieht im Falle, dass die Kopplungsmittel als Rastnasen ausgebildet sind, besonders durch gezieltes Abbrechen der Rastnase an dem Segment, für den Fall, dass die Kopplungsmittel als Querkoppelelement ausgebildet sind, durch Herausnehmen des Querkoppelelements an dem Segment.

In bevorzugter Ausgestaltung ist das Kopplungsmittel an der Stützschiene mindestens eine Rastnase an mindestens einem Segment der einen Stützschiene, welches zum Eingriff in eine Nut an einem Segment der benachbarten Stützschiene ausgebildet ist. Alternative form- und/oder kraftschlüssige Kopplungsmittel sind dem Fachmann bekannt. Neben Rastnasen, welche in Nuten eingreifen, sind auch Druckknöpfe, rastende Stifte sowie Stifte und Nägel mit Klemmsitz sowie Nieten, Schrauben und ähnliches bevorzugt.

In einer besonderen Ausgestaltung ist als Kopplungsmittel mindestens eine Führung an mindestens einem Segment der einen Stützschiene ausgebildet, welche zur Aufnahme eines separaten Querkoppelelements dient. Das Querkoppelelement wird mechanisch so in der Führung geführt, dass es in eine entsprechende Führung an einem Segment der benachbarten Stützschiene eingreift. Ein solches Querkoppelelement ist beispielsweise in Form eines steifen Streifens oder Drahts aus Metall oder Kunststoff gebildet. In dieser besonderen Ausgestaltung ist es besonders möglich, gezielt zwei oder mehrere nebeneinander liegende Schienen mittels eines Querkoppelelements zu verkoppeln. Dabei kann die Auswahl der quer zu verkoppelnden Stützschienen durch einfache Wahl der Länge des einzusetzenden Querkoppelelements bestimmt werden. Besonders sind die Führungen an dem mindestens einen Segment zur Aufnahme des Querkoppelelements in Form von auf den Grundkörper der Stützschiene aufgesetzten Bügeln ausgebildet.

In einer besonderen Ausgestaltung der Erfindung als Handorthese ist daher vor allem vorgesehen, dass an einem insbesondere als Handauflage dienenden Grundkörper, welcher in an sich bekannter Weise am Unterarm befestigt werden kann, eine oder vor allem mehrere der erfindungsgemäßen Stützschienen ausgebildet sind und/oder mit dem Grundkörper mechanisch kraft- und/oder formschlüssig, insbesondere reversibel, verbunden sind. Je nach medizinischer Indikation kann also an dem Grundkörper eine oder mehrere der erfindungsgemäßen Stützschienen ausgebildet oder angebracht sein, um einen oder mehrere Finger der Hand zu stützen und dabei ein oder mehrere der Fingerglieder jeweils eines Fingers zu stützen oder zu fixieren. Die Erfindung bietet nun die Möglichkeit, mehrere benachbarte Stützschienen über die erfindungsgemäß vorgesehen Querkopplungsmittel miteinander mechanisch zu verkoppeln, um eine orthopädisch zweckmäßige, individuell angepasste Stützwirkung für die Fingergelenke zu erzielen.

Die Materialausdünnung oder Kerbung an der Segmentgrenze dient zusätzlich oder alternativ dazu, ein Kürzen der Stützschiene zur individuellen Anpassung der Stützfunktion je nach medizinischer Indikation durch den Anwender, den Arzt und insbesondere durch den Orthopädietechniker in einfacher Weise zu ermöglichen. Die Materialausdünnung dient somit als "Sollbruchstelle" und/oder als vorgegebener Ansatz für ein Schneidwerkzeug. In dieser Ausgestaltung der Erfindung kann, je nach medizinischer Indikation, jede zunächst in ihrer Grundform für alle Körperglieder, insbesondere die Finger, gleich ausgebildete Stützschiene individuell gekürzt werden, um die Stützwirkung gezielt auf einzelne Körpergelenke, insbesondere auf individuelle Fingergelenke einzelner Finger zu beschränken. In der ungekürzten Grundform dient die Stützschiene, beispielsweise in der Ausgestaltung als Fingerschiene also der Stützung sämtlicher Fingerglieder eines Fingers, in der gekürzten Form, der Stützung der Fingermittelglieds und des -grundglieds, in der weiter gekürzten Form nur der Stützung des Fingergrundgelenks. Weiter kann die für einen Mittelfinger dimensionierte Grundform der Fingerschiene durch einfaches Kürzen an der durch die Segmentierung vorgegebenen Position in eine verkürzte Variante, die für den kleinen Finger passt, umgebaut werden. Auf diese Weise lässt sich eine Fingerorthese aus gleichartigen Stützschienen individuell zusammenstellen, was nicht nur die Handhabbarkeit verbessert und das orthopädische Anwendungsspektrum vergrößert, sondern auch den Fertigungsaufwand der Orthese ressourcenschonend vermindert.

Demgemäß betrifft die Erfindung auch eine Handorthese, welche die Gelenkstütze, besonders mindestens eine, insbesondere zwei quer verkoppelbare Stützschienen, welche jeweils in versteifbare Segmente strukturiert sind, aufweist. Dabei sind die Knochenglieder die Fingerknochen.

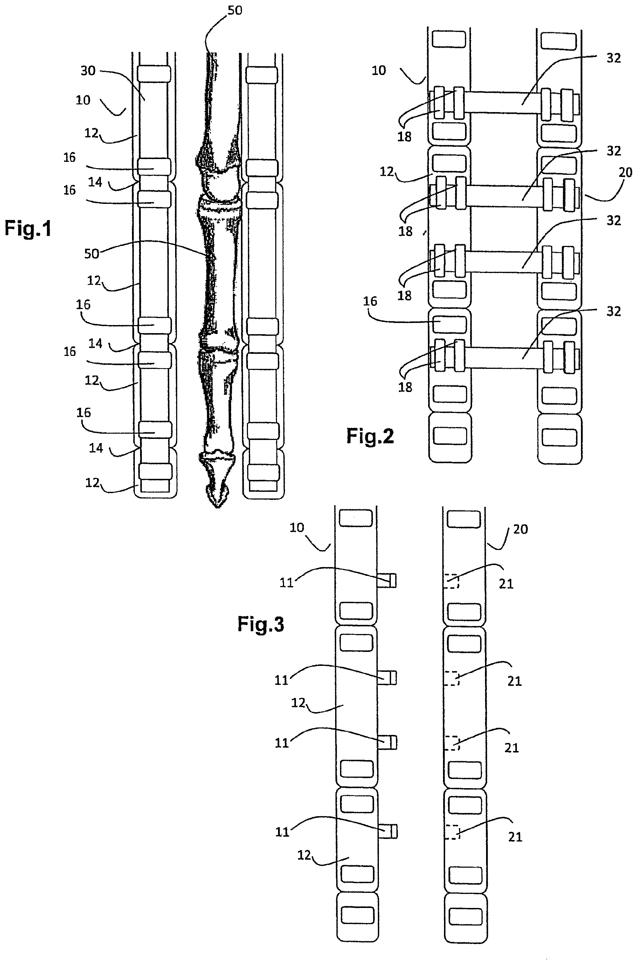

Figur 1A zeigt einen schematischen Aufbau der erfindungsgemäßen Stützschiene 10 der Gelenkstütze, wobei die Stützschiene in Segmente 12 strukturiert ist. Weiter weist die Stützschiene Kerben oder Materialverdünnungen 14 auf, die der Strukturierung der Stützschiene in mindestens zwei Segmente 12 dienen. Die Segmente 12 sind an die zu stützenden gelenkig verbundenen Knochenglieder 50 anpassbar, wobei ein Segment 12 einem bestimmten Knochenglied 50 zuordenbar ist. Weiter weisen die Segmente der Stützschiene mindestens eine Führung 16 auf, worin ein Versteifungselement 30 aufgenommen sein kann, welches die durch die Kerbe oder Materialverdünnung 14 gebildete flexible Verbindung zwischen zwei Segmenten 12 mechanisch versteift werden kann. Figur 1B zeigt beispielhaft die Zuordnung der einzelnen Segmente 12 zu jeweils einem Knochenglied 50 am Beispiel eines Fingers der Hand.
Figur 2 zeigt zwei jeweils gleichartig aufgebaute Stützschienen, welche mittels Kopplungsmittel 18 in Verbindung mit 32 mechanisch querverkoppelt sind. In der Ausführung nach Figur 2 sind die Kopplungsmittel als Führungen 18 ausgebildet, worin ein Querkopplungselement 32 geführt ist, was mit entsprechenden Führung 18 der benachbarten Stützschiene 20 in Eingriff kommt. Besonders ist vorgesehen, pro Segment mindestens ein separates Kopplungsmittel zur Querkopplung vorzusehen. Wobei selektiv segmentweise die Querverkopplung hergestellt werden kann. Die Segmentierung der Stützschiene wird also erfindungsgemäß bevorzugt durch konstruktive Maßnahmen wie Einkerbung im Bereich der Segmentgrenzen und/oder alternativ durch Materialausdünnung im Bereich der Segmentgrenzen ermöglicht. Bevorzugt sind die mindestens zwei Segmente einer Stützschiene also durch Einkerbung und/oder Materialausdünnung an der Schiene voneinander abgesetzt beziehungsweise getrennt. Bevorzugt dient dabei die Einkerbung und/oder Materialausdünnung in der Art eines Foliengelenks der Beweglichkeit der Stützschiene, sodass diese der Bewegung der von ihr zu stützenden gelenkig verbundenen Knochengliedern folgen kann, um diese mechanisch zu unterstützen.
Figur 3 zeigt eine alternative Ausgestaltung des Kopplungsmittels zur mechanischen Querverkopplung benachbarter Stützschienen. An einer Stützschiene 10 sind segmentweise Rastnasen 11 ausgebildet, welche mit Nuten 21 einer benachbarten Stützschiene 20 in Eingriff kommen können, um die mechanische Querverkopplung zu bewirken.
   Besonders kann vorgesehen sein, dass zum Zwecke des einfachen modularen Aufbaus Stützschiene 10 und benachbarte Stützschiene 20 gleichartig aufgebaut sind, wonach sowohl Stützschiene 10 und Stützschiene 20 jeweils Rastnasen 11 und Nuten 21 aufweist. Zur Übersicht sind für Stützschiene 10 nur die Rastnasen 11, für Stützschiene nur die Nuten 21 zur Aufnahme der Rastnasen gezeigt.

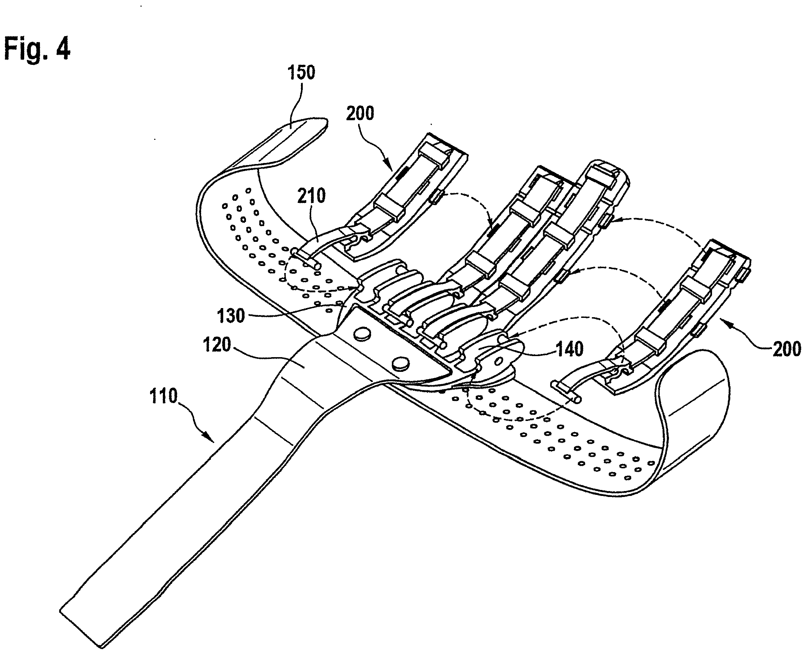

Die Figuren 4 bis 8 zeigen eine besondere Ausgestaltung der erfindungsgemäßen Gelenkstütze zur Anwendung als Finger-/Handorthese. Die Erfindung ist nicht auf diese Anwendung beschränkt. Sie zeigt vielmehr beispielhaft das Grundprinzip der erfindungsgemäßen modular aufgebauten Gelenkstütze. Dabei zeigt Figur 4 einen Grundkörper 110, welcher als Handauflage dient, bestehend aus einer Aufnahme 130 zur Aufnahme mindestens einer Stützschiene 200 über mechanische Rastkopplungen 210 an der Stützschiene 200, die mit Gegenstücken 140 der Aufnahme 130 des Grundkörpers 110 in Eingriff kommen können. Weiter weist der Grundkörper 110 eine Schiene 120 und mindestens eine Vergurtung 150 auf, um den Grundkörper sicher zu fixieren. Die Ausgestaltung der Fixierung des Grundkörpers, das heißt der Handauflage am Unterarm, ist hier zur Vereinfachung nur schematisch dargestellt. Der Fachmann kennt geeignete Maßnahmen zur sicheren Fixierung einer Handauflage am Unterarm. Alternativ zu der dargestellten mindestens einen Vergurtung 150 kann der Grundkörper 110 mittels der Schiene 120 in eine an sich bekannte Mittelhand-/Unterarmorthese eingesteckt und dort fixiert werden.

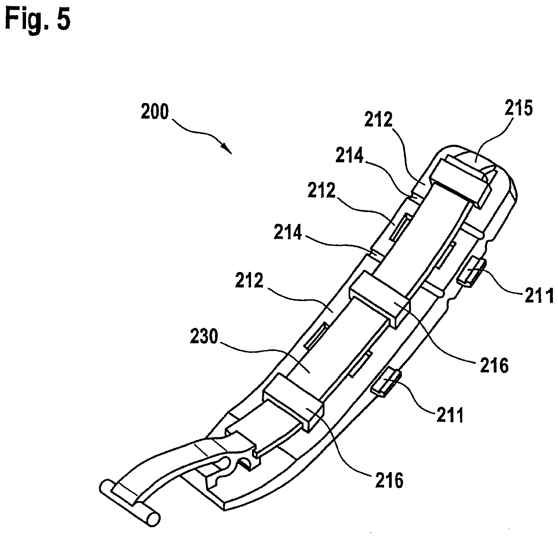

Die Figur 5 zeigt eine schematische Darstellung einer Ausgestaltung der erfindungsgemäßen Gelenkstütze, wie sie für die Anwendung an einer Finger-/Handorthese, insbesondere zum modularen Aufbau geeignet ist. Die Stützschiene 200 teilt sich über Materialverdünnungen 214 in die Segmente 212. Diese sind in ihrer Dimensionierung an die Größe des zu stützenden Fingers angepasst. An Segmenten 212 sind Führungen 216 in Form von Brücken zur Aufnahme eines Versteifungselements 230 ausgebildet, um eine mechanische Versteifung der Stützschiene 200 über Segmentgrenzen hinweg zu ermöglichen. Zur Fixierung des Versteifungselements 230 sind an mindestens dem Endelement federnde Rastungen 215 vorgesehen, um ein Herausgleiten des eingesetzten Versteifungselements 230 aus der Stützschiene zu verhindern.

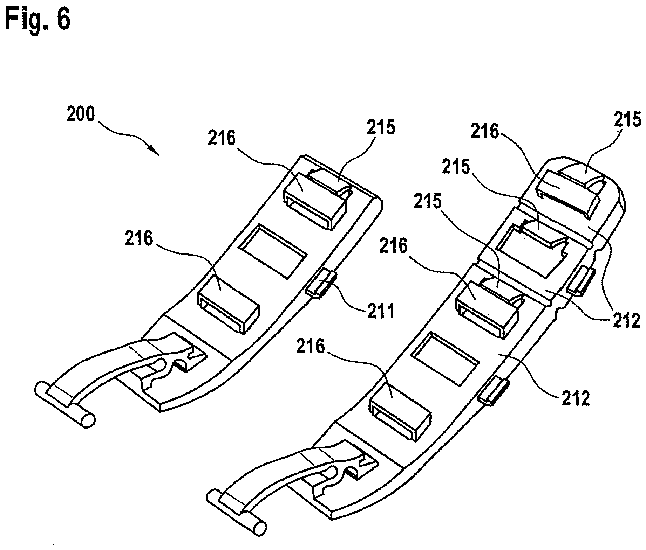

Die Figur 6 zeigt die Stützschiene 200 in ihrer individuellen Anpassbarkeit der Länge zur Steuerung ihrer Stützfunktion. Durch die Materialverdünnungen 214 kann an den Grenzen zwischen den Segmenten 212 geschnitten werden. Um dennoch den sicheren Sitz eines Versteifungselements 230 an der Stützschiene 200 zu ermöglichen, sind in jedem potentiellen Endsegment 212 federnde Rastungen 215 vorgesehen, welche mit dem einzusetzenden Versteifungselement 230 in Eingriff kommen können. Diese Figur zeigt besonders den modularen Aspekt der erfindungsgemäßen Gelenkstütze: Nicht nur benachbarte Stützschienen sind gleichartig aufgebaut, auch die besondere Ausgestaltung der Segmente an einer Stützschiene, besonders in Verbindung mit den federnden Rastungen 215 ermöglicht die individuelle Anpassung und Ausgestaltung der Finger-/Handorthese, ausgehend von einer einzigen gleichartig aufgebauten Stützschiene. Diese kann insbesondere als Spritzgussteil in einer einzigen Form mehrfach produziert werden.
   In der Ausgestaltung nach Figur 6 weisen die Stützschienen 200 Rastnasen 211 auf, die in bevorzugt an derselben Stützschiene gegenüberliegend ausgebildeten Nuten (in der Figur nicht gezeigt) in Eingriff kommen können, um die Querverkopplung mindestens zweier Stützschienen untereinander zu ermöglichen.

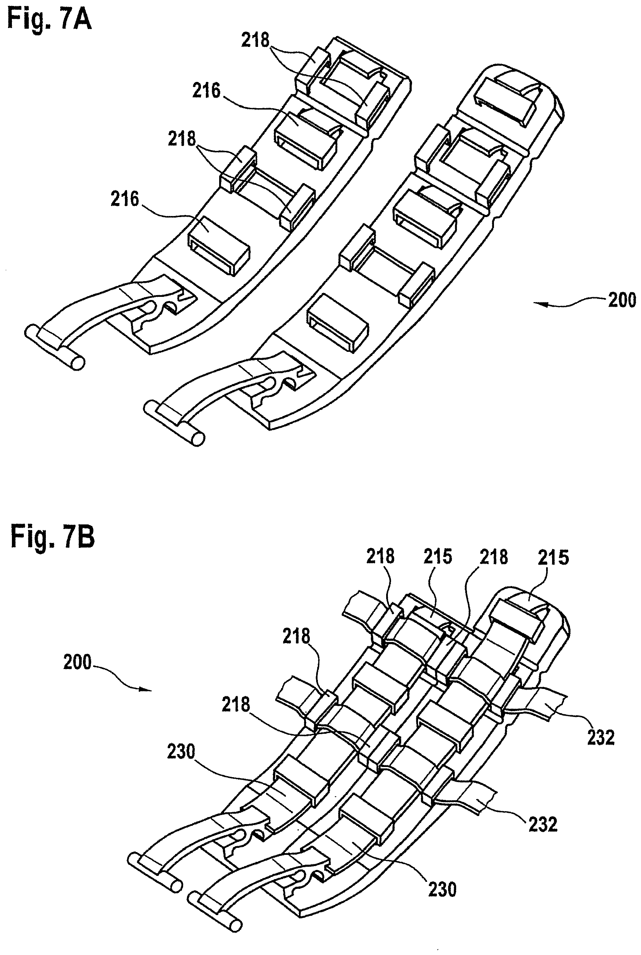

In den Figuren 7A und 7B sind Stützschienen 20 mit einer alternativen erfindungsgemäßen Querkupplung dargestellt. Als Kopplungsmittel dienen hier an den Segmenten 212 angesetzte Bügel 218, welche Querkoppelelemente 232, besonders in Form von unelastischen Metall- oder Plastikstreifen, aufnehmen können, um mindestens zwei benachbarte Stützschienen 200 miteinander zu verkoppeln. Besonders ist vorgesehen, die Koppelmittel zur Querverkopplung der Stützschienen segmentweise vorzusehen.

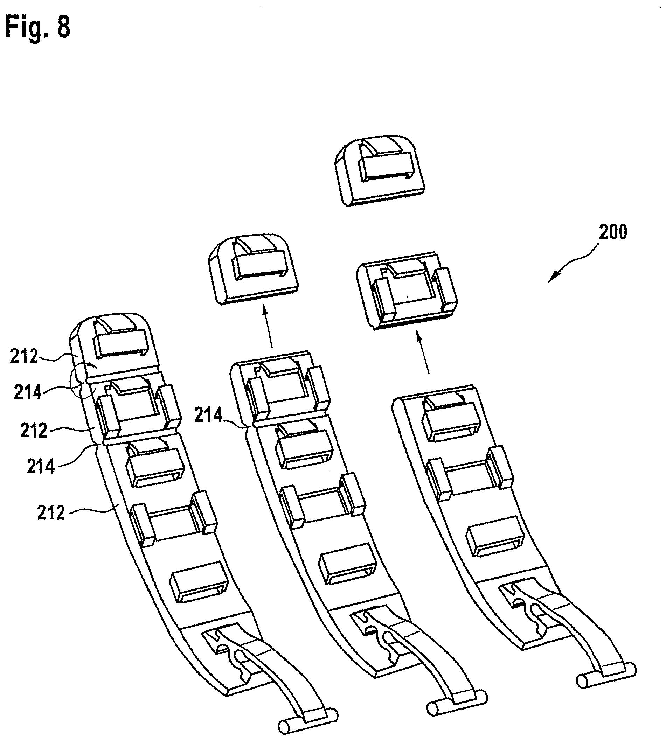

Die Figur 8 illustriert schematisch die Funktion der Materialausdünnung 214 an den Segmentgrenzen. Sie dient zum einen der gelenkigen Verbindung der Segmente 212 im Sinne eines Foliengelenks. Zum anderen dienen sie als "Sollbruchstelle", um ein Einkürzen der Stützschiene je nach gewünschter Stützfunktion zu ermöglichen.

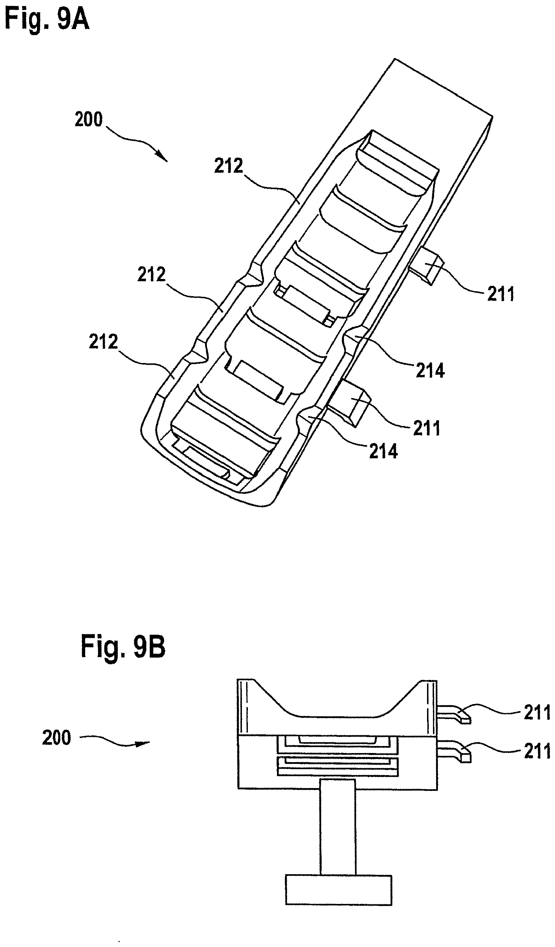

Die Figuren 9A und 9B zeigen eine Draufsicht (Figur 9A) und eine Seitenansicht (Figur 9B) der Stützschiene, um eine weitere bauliche Ausgestaltung in Zusammenhang der erfindungsgemäßen Gelenkstütze zur Verwendung in einer Finger-/Handorthese zu ermöglichen: Die Gelenkschiene bildet insgesamt eine Wanne, worin der zu stützende Finger geführt wird. Die Materialverdünnungen 214 sind hierbei besonders als Kerben ausgebildet, welche die versteifende Funktion des Wannenrands unterdrücken und die Flexibilität und/oder Abtrennbarkeit zwischen den Segmenten 212 zu ermöglichen.

## Patentansprüche

1. Gelenkstütze zur Stützung gelenkig verbundener Knochenglieder (50), mit mindestens einer Stützschiene (10), welche in mindestens zwei Segmente (12) strukturiert ist, wobei die Stützschiene (10) je Segment (12) mindestens eine Führung (16) zur Aufnahme eines Versteifungselements (30) zur Längskopplung der mindestens zwei Segmente (12) aufweist, **dadurch gekennzeichnet, dass** die Stützschiene (10) Kopplungsmittel (11,21,18,32) zur Querkopplung dieser Stützschiene (10) mit mindestens einer weiteren, dazu benachbarten Stützschiene (20) aufweist.

2. Gelenkstütze nach Anspruch 1, enthaltend mindestens zwei miteinander quer verkoppelbare gleichartige Stützschienen (10,20).

3. Gelenkstütze nach Anspruch 1 oder 2, wobei als Kopplungsmittel mindestens eine Rastnase (11) an mindestens einen Segment (12) der einen Stützschiene (10) zum Eingriff in eine Nut (21) an einem Segment (12) der benachbarten Stützschiene (20) ausgebildet ist.

4. Gelenkstütze nach Anspruch 1 oder 2, wobei als Kopplungsmittel eine Führung (18) an mindestens einem Segment (12) der einen Stützschiene (10) zur Aufnahme eines Querkoppelelements (32), zum Eingriff in eine Führung (18) an einem Segment (12) der benachbarten Stützschiene (20) ausgebildet ist.

5. Gelenkstütze nach Anspruch 4, wobei die Führung zur Aufnahme eines Querkoppelelements (32) als mindestens ein Bügel (18) auf der Stützschiene (10) ausgebildet ist.

6. Gelenkstütze nach einem der vorstehenden Ansprüche, wobei die Führung zur Aufnahme eines Versteifungselements (30) als mindestens ein Bügel (16) auf der Stützschiene (10) ausgebildet ist.

7. Gelenkstütze nach einem der vorstehenden Ansprüche, wobei die Führung zur Aufnahme eines Versteifungselements (30) als Kanal innerhalb der Stützschiene (10) ausgebildet ist.

8. Gelenkstütze nach einem der vorstehenden Ansprüche, wobei die Segmente (12) durch Einkerbung und/oder Materialausdünnung (14) an der Schiene (10) voneinander getrennt sind.

9. Handorthese, enthaltend die Gelenkstütze nach einem der Ansprüche 1 bis 8, wobei die Knochenglieder (50) Fingerknochen sind.

## Claims

1. A joint orthosis for supporting articulated bone members (50) comprising at least one support splint (10) structured into at least two segments (12), the support splint (10) having at least one guide (16) per segment (12) to receive a stiffening element (30) for longitudinal coupling of the at least two segments (12), **characterized in that** the support splint (10) comprises coupling means (11, 21, 18, 32) for transverse coupling of said support splint (10) to at least one further and neighbouring support splint (20).

2. The joint orthosis according to claim 1, comprising at least two similar support splints (10, 20) that can be coupled transversely to one another.

3. The joint orthosis according to claim 1 or 2, wherein at least one detent (11) is formed on at least one segment (12) of the one support splint (10) as coupling means for engagement in a groove (21) on a segment (12) of the neighbouring support splint (20).

4. The joint orthosis according to claim 1 or 2, wherein a guide (18) is formed at on at least one segment (12) of the one support splint (10) as coupling means to receive a transverse coupling element (32) for engagement in a guide (18) on a segment (12) of a neighbouring support splint (20).

5. The joint orthosis according to claim 4, wherein the guide to receive a transverse coupling element (32) is designed as at least one frame (18) on the support splint (10).

6. The joint orthosis according to any one of the preceding claims, wherein the guide is designed to receive a stiffening element (30) as at least one frame (16) on the support splint (10).

7. The joint orthosis according to any one of the preceding claims, wherein the guide is designed as a channel within the support splint (10) to receive a reinforcing element (30).

8. The joint orthosis according to any one of the preceding claims, wherein the segments (12) are separated from one another by notching and/or areas of thinner material (14) on the splint (10).

9. A hand orthotic device, comprising the joint orthosis according to any one of claims 1 to 8, wherein the bone members (50) are finger bones.

## Revendications

1. Support articulaire pour le support de membres osseux reliés de manière articulée (50), avec au moins un rail de support (10) qui est structuré en au moins deux segments (12), dans lequel le rail de support (10) présente par segment (12) au moins un guide (16) pour la réception d'un élément de renforcement (30) pour l'accouplement longitudinal des au moins deux segments (12), **caractérisé en ce que** le rail de support (10) présente des moyens d'accouplement (11, 21, 18, 32) pour l'accouplement transversal de ce rail de support (10) avec au moins un autre rail de support (20) qui lui est voisin.

2. Support articulaire selon la revendication 1, contenant au moins deux rails de support de même type (10, 20), pouvant être accouplés l'un à l'autre de manière transversale.

3. Support articulaire selon la revendication 1 ou 2, dans lequel au moins un nez d'encliquetage (11) est réalisé en tant que moyen d'accouplement sur au moins un segment (12) d'un des rails de support (10) pour l'engrènement dans une rainure (21) sur un segment (12) du rail de support voisin (20).

4. Support articulaire selon la revendication 1 ou 2, dans lequel un guide (18) est réalisé en tant que moyen d'accouplement sur au moins un segment (12) d'un des rails de support (10) pour la réception d'un élément d'accouplement transversal (32) pour l'engrènement dans un guide (18) sur un segment (12) du rail de support voisin (20).

5. Support articulaire selon la revendication 4, dans lequel le guide pour la réception d'un élément d'accouplement transversal (32) est réalisé en tant qu'au moins un arceau (18) sur le rail de support (10).

6. Support articulaire selon une des revendications précédentes, dans lequel le guide pour la réception d'un élément de renforcement (30) est réalisé en tant qu'au moins un arceau (16) sur le rail de support (10).

7. Support articulaire selon une des revendications précédentes, dans lequel le guide pour la réception d'un élément de renforcement (30) est réalisé en tant que canal au sein du rail de support (10).

8. Support articulaire selon une des revendications précédentes, dans lequel les segments (12) sont séparés l'un de l'autre par rainurage et/ou amincissement de matériau (14) sur le rail (10).

9. Orthèse de main contenant le support articulaire selon une des revendications 1 à 8, dans laquelle les membres osseux (50) sont des os digitaux.
